# EUROPÄISCHE PATENTSCHRIFT

(11) **EP 3 620 126 B1**
(45) Veröffentlichungstag und Bekanntmachung des Hinweises auf die Patenterteilung: **23.12.2020**
(21) Anmeldenummer: 19205805.5
(22) Anmeldetag: 28.06.2017
(51) Int. Cl.: A61B 17/70, A61B 17/86, A61B 90/00

(54) **POLYAXIALPEDIKELSCHRAUBE**
POLYAXIAL PEDICLE SCREW
VIS PÉDICULAIRE POLYAXIALE

(30) Priorität: 02.08.2016 DE 102016114266
(43) Veröffentlichungstag der Anmeldung: 11.03.2020
(62) Teilanmeldung aus: 17733833.2
(73) Patentinhaber: Silony Medical International AG, 8500 Frauenfeld (CH)
(72) Erfinder: Heuer, Frank, 70794 Filderstadt (DE)
(74) Vertreter: DREISS Patentanwälte PartG mbB

(56) Entgegenhaltungen:
- EP-A2- 2 638 874
- EP-B1- 2 502 594
- WO-A1-2015/155702

## Beschreibung

Die Erfindung betrifft eine Polyaxialpedikelschraube mit einem einen Gewindeschaft und einen Kopf aufweisenden Schraubanker und mit einem in einer Seitenansicht U-förmigen und eine Aufnahmeöffnung für ein Korrekturelement, insbesondere einen Korrekturstab, aufweisenden Gabelkopf mit zwei Schenkeln, wobei der Kopf des Schraubankers in einem distalen Endbereich des Gabelkopfs polyaxial verschwenkbar gelagert ist und der Gabelkopf in einer vom Chirurgen intendierten Schwenkstellung bezüglich des Kopfs des im Knochen festgelegten oder festlegbaren Schraubankers fixierbar ist, wobei der Gabelkopf eine axiale Richtung und eine hierzu radiale Richtung aufweist sowie ein distales Ende benachbart zu dem Schraubanker und ein hiervon in axialer Richtung abgewandtes proximales Ende aufweist, so dass auch eine distale Richtung und eine proximale Richtung definiert ist, wobei sich die Schenkel ausgehend von einem distalen Bereich des Gabelkopfs in proximaler Richtung erstrecken und proximal frei enden und zwischen sich die Aufnahmeöffnung für das Korrekturelement begrenzen, wobei die Schenkel einen radial äußeren Umfangsbereich aufweisen, in dem zum Ergreifen des Gabelkopfs mittels eines Handhabungsinstruments wenigstens eine Haltenut oder eine sonstige Instrumentenansetzstelle ausgebildet ist, und mit einem im Gabelkopf zwischen dem Kopf des Schraubankers und dem Korrekturelement anordenbaren Druckstück, das einerseits auf dem Kopf des Schraubankers aufsitzt und andererseits durch das Korrekturelement belastbar ist, wobei auf das Druckstück mittels des am Gabelkopf angreifenden Handhabungsinstruments oder mittels eines anderen Instruments eine temporär wirkende Kraft in Richtung auf den Kopf des Schraubankers ausübbar ist, so dass der Gabelkopf hierdurch gegenüber dem Kopf des Schraubankers temporär in einer vom Chirurgen gewünschten Schwenkstellung unverstellbar gehalten ist, während das Korrekturelement bewegbar bleibt, solange bis das Korrekturelement, der Gabelkopf, das Druckstück und der Kopf des Schraubankers in einer gewünschten Lage und Stellung zueinander durch ein anderes Stellmittel dauerhaft gegeneinander fixiert werden, wobei das Druckstück im Bereich eines Schenkels des Gabelkopfs über ein Schwenklager gegen diesen Schenkel in axialer Richtung abgestützt ist.

Eine derartige Pedikelschraube ist bekannt aus WO 2015/155702 A1. Bei dieser bekannten Pedikelschraube dient das Schwenklager aber lediglich als Verliersicherung für das durch eine ausladende Queröffnung im Gabelkopf seitlich eingeführte Druckstück. Das Druckstück selbst wird nämlich beim bestimmungsgemäßen Betrieb nicht verschwenkt, sondern das Schwenklager ist derart spielbehaftet, dass das Druckstück durch zwei seitliche Stößel eines Instruments in axialer Richtung, also nur translatorisch, gegen den Kopf des Schraubankers gezwungen wird. Auch bei EP 2 638 874 A2 ist eine ausladende seitliche Queröffnung im Gabelkopf vorgesehen, um sowohl den Schraubanker als auch das Druckstück von der Seite einsetzen zu können. Auch hier ist das Druckstück über eine Schwenklagerung als Verliersicherung im Gabelkopf gehalten. Eine temporäre Fixierbarkeit des Druckstücks ist nicht möglich. Weitere Pedikelschrauben sind bekannt, insbesondere aus EP 2 502 594 B1 oder aus US 2014/0236236 A1.

Bei einer weiteren Pedikelschraube gemäß US 2010/0262196 A1 weist das Druckstück an seinem Außenumfang eine in axialer Richtung erstreckte Nut zur Aufnahme eines in radialer Richtung durch den Gabelkopf hindurch nach innen erstreckten Stifts auf. Hierdurch soll eine verliersichere Ausrichtung des Druckstücks innerhalb des Gabelkopfs realisiert werden.

Der vorliegenden Erfindung liegt die Aufgabe zugrunde, eine Polyaxialpedikelschraube der eingangs genannten Art zu schaffen, bei der der freizuhaltende Zugangsraum bei dem Gabelkopf möglichst gering ist und bei der eine hohe Stellkraft und damit eine sichere temporäre Fixierung realisierbar ist.

Diese Aufgabe wird bei einer Polyaxialpedikelschraube der eingangs genannten Art erfindungsgemäß dadurch gelöst, dass eine in axialer Richtung wirkende Stellkraft nur an einer einzigen Stelle dem Schwenklager diametral gegenüberliegend in das Druckstück eingeleitet wird, indem das Druckstück dem Schwenklager diametral gegenüberliegend einen Aufnahmebereich für die in axialer Richtung wirkende Stellkraft aufweist, welche das Druckstück in distaler Richtung bezüglich des Schwenklagers zu verschwenken sucht und hierdurch die temporär wirkende Kraft in Richtung auf den Kopf des Schraubankers bewirkt, und dass der Gabelkopf hierfür eine Zugangsausnehmung in genau einem der Schenkel aufweist, durch welche Zugang auf den Aufnahmebereich des Druckstücks mittels des Handhabungsinstruments oder des anderen Instruments genommen werden kann.
Dadurch, dass das Druckstück erfindungsgemäß geringfügig schwenkbar innerhalb des Gabelkopfs vorgesehen ist, ist es ausreichend, wenn die in axialer Richtung wirkende Stellkraft nur an einer einzigen Stelle dem Schwenklager diametral gegenüberliegend in das Druckstück eingeleitet wird. Im Unterschied zu den vorbekannten Pedikelschrauben muss daher nur mittels eines einzigen Stellorgans, insbesondere in Form einer Schubstange, eines Schubstößels oder -stempels oder dergleichen, an einer einzigen Stelle des Druckstücks angegriffen zu werden. Der hierfür erforderliche Zugangsraum für das Stellorgan ist gegenüber dem Stand der Technik reduziert. Ein weiterer wesentlicher Vorteil ist darin zu sehen, dass das Druckstück gewissermaßen als Klemmhebel fungiert. Daher wird die in axialer Richtung auf den Aufnahmebereich des Druckstücks wirkende Stellkraft nach den Hebelgesetzen verstärkt, etwa wie bei einem zwei Klammerschenkel aufweisenden Nussknacker.

Nach einer vorteilhaften und einfach zu realisierenden Ausführungsform der Erfindung weist das Schwenklager ein in den Gabelkopf eingesetztes langgestrecktes Wellenelement auf, das in einer Ebene orthogonal zur axialen Richtung des Gabelkopfs erstreckt ist und gegen welches das Druckstück in axialer Richtung abgestützt ist. Das Wellenelement kann von einem Stift gebildet sein, der in eine Aufnahmeöffnung des Gabelkopfs von außen einsteckbar ist.

Im Betriebszustand ist das langgestreckte Wellenelement vorzugsweise ungefähr parallel zu dem in den Gabelkopf eingesetzten Korrekturelement erstreckt. Es erweist sich als vorteilhaft, wenn das langgestreckte Wellenelement an seinen Enden innerhalb der Wandung des Gabelkopfs aufgenommen ist und mit einem mittleren Abschnitt in den von den Schenkeln des Gabelkopfs begrenzten Innenraum, also nach radial innen einragt.

Weiter kann es sich als vorteilhaft erweisen, wenn das Schwenklager ein radial innen am Gabelkopf ausgebildetes Auflager umfasst, gegen welches das Druckstück in axialer Richtung abgestützt ist. Dieses Auflager kann gewissermaßen punktuell ausgebildet sein, oder es kann eine Erstreckung quer zur Schwenkebene von 0,5 - 5 mm, insbesondere von 0,5 - 3 mm haben.
Es kann sich als vorteilhaft erweisen, wenn dieses Auflager von einem freien Ende eines von außen nach radial innen durch einen Schenkel des Gabelkopfs gesteckten Stifts gebildet ist.

Nach einer anderen Ausführungsform kann es sich als vorteilhaft erweisen, wenn das Auflager von einer am Gabelkopf, insbesondere einstückig mit dem Gabelkopf ausgebildeten Stufe oder Nutflanke gebildet ist. Solchenfalls kann das Auflager bei der Formgebung des Gabelkopfs hergestellt werden, ohne dass ein weiteres Teil erforderlich ist.

Zur Ausbildung des Schwenklagers zwischen dem Druckstück und dem Gabelkopf erweist es sich als vorteilhaft, wenn das Schwenklager eine Ausnehmung in dem Druckstück umfasst. In diese Ausnehmung kann dann zur Bildung der Schwenklagerstelle ein Wellenelement oder ein sonstiges Auflager, insbesondere in Form eines Stifts oder Stiftendes eingreifen.

Es kann sich auch als vorteilhaft erweisen, wenn das Schwenklager einen radial vorstehenden Stützabschnitt an dem Druckstück umfasst, mittels dessen das Druckstück am Gabelkopf axial abgestützt und dabei schwenkbar ist.

Es wäre denkbar, dass der Aufnahmebereich des Druckstücks für die in axialer Richtung wirkende Stellkraft von einem distalen Ende des Druckstücks gebildet ist, ohne dass die reguläre Form des Druckstücks zur Ausbildung des Aufnahmebereichs verändert wird. Demgegenüber erweist sich als vorteilhaft, wenn der Aufnahmebereich des Druckstücks für die in axialer Richtung wirkende Stellkraft von einem radialen Ansatz oder Fortsatz des Druckstücks gebildet ist. Durch diese Maßnahme, kann der Aufnahmebereich des Druckstücks weiter nach radial außen verlagert werden, was die durch Hebelwirkung auf den Kopf des Schraubankers einwirkende Kraft weiter verstärkt. Der genannte radiale Ansatz oder Fortsatz kann einstückig mit dem Druckstück ausgebildet sein, oder er kann als separates Element an das Druckstück angefügt sein.

Insbesondere wenn der Aufnahmebereich von einem radialen Ansatz oder Fortsatz des Druckstücks gebildet ist, erweist es sich als vorteilhaft, wenn der Gabelkopf eine Ausnehmung für den Aufnahmebereich des Druckstücks aufweist. Durch diese Ausnehmung kann sich der Aufnahmebereich des Druckstücks nach radial außen erstrecken.

Diese Ausnehmung für den Aufnahmebereich des Druckstücks kann in axialer Richtung oder in Umfangsrichtung an einem Schenkel des Gabelkopfs jeweils innen erstreckt und jeweils nach innen öffnend ausgebildet sein. Beispielsweise könnte die Ausnehmung durch einen Innengewindeabschnitt des Schenkels hindurch in axialer Richtung erstreckt sein, so dass das Druckstück von oben in den Gabelkopf einführbar ist, wobei der Fortsatz in die Ausnehmung radial einragt. Es kann sich aber auch als vorteilhaft erweisen, wenn die Ausnehmung ausgehend von einer seitlichen Flanke des Schenkels in Umfangsrichtung oder quer zur axialen Richtung erstreckt ist. Solchenfalls kann der Ansatz oder Fortsatz des Druckstücks in diese Ausnehmung eingedreht werden.

Weiter erweist sich als vorteilhaft, wenn die Ausnehmung in radialer Richtung durch einen Wandbereich eines Schenkels des Gabelkopfs hindurchgehend ausgebildet und erstreckt ist. Somit gelangt der Ansatz oder Fortsatz des Druckstücks durch die Ausnehmung nach radial außen, so dass dessen Aufnahmebereich für die Stellkraft radial außen exponiert wird.

Vorzugsweise ist die Ausnehmung ausgehend von einer Flanke des Schenkels des Gabelkopfs zuerst in Umfangsrichtung oder quer zur axialen Richtung innen an dem Schenkel und dann in radialer Richtung durch einen Wandbereich des Schenkels hindurchgehend ausgebildet und erstreckt. Hierdurch ist eine handhabungsfreundliche Montage des Druckstücks am Gabelkopf möglich, indem das Grundstück in axialer Richtung eingesetzt und dann um die axiale Richtung verdreht wird bis der Aufnahmebereich des Druckstücks in die radiale Ausnehmung eingreift oder durch diese hindurchgreift. Insbesondere ist es denkbar und vorteilhaft, wenn die Ausnehmung in radialer Richtung durch einen Schenkel des Gabelkopfs ganz hindurcherstreckt ist, also in dem Außenumfang des Schenkels ausmündet.

Es erweist sich als zweckmäßig, wenn die Zugangsausnehmung ausgehend von einem proximalen Ende des Schenkels des Gabelkopfs in axialer Richtung erstreckt ist, da die axiale Richtung die typische Zugriffsrichtung auf die Pedikelschraube darstellt.

In Weiterbildung dieses Erfindungsgedankens ist es denkbar und vorteilhaft, wenn die Zugangsausnehmung radial innen ausgebildet ist und nach radial innen offen ist oder radial außen ausgebildet ist und nach radial außen offen ist oder als axiale Bohrung in dem Schenkel ausgebildet ist.

Wenn die Zugangsausnehmung als axiale Bohrung in dem Schenkel ausgebildet ist, so kann die axiale Bohrung ein Innengewinde tragen, in die das andere Instrument mit einem Gewindestößelabschnitt einschraubbar ist, so dass der Gewindestößelabschnitt mit seinem distalen Ende gegen den Aufnahmeabschnitt des Druckstücks festziehbar ist.
Bei allen Ausführungsformen erweist es sich als vorteilhaft, wenn das Schwenklager ein axiales Spiel aufweist, derart, dass das Druckstück bei Krafteinleitung in axialer Richtung über das Korrekturelement eine axiale Stellbewegung ausführen kann, ohne dass diese Stellbewegung durch das Schwenklager behindert würde. Auf diese Weise kann bei der schlussendlichen dauerhaften Fixierung der Komponenten der Polyaxialpedikelschraube verhindert werden, dass innerhalb des Druckstücks Zugspannungen oder Verwindungsspannungen auftreten, die durch eine scharnierhafte Anlenkung des Druckstücks bedingt sein könnten. Dadurch, dass die gegeneinander abgestützten Bereiche des Druckstücks und des Gabelkopfs, welche das Schwenklager bilden, mit einem axialen Spiel versehen sind, kann einerseits eine Schwenklagerung zur Ausbildung der temporären Klemmkraft und andererseits eine gewisse axiale Verstellbarkeit für die schlussendliche dauerhafte Klemmung und Fixierung der Komponenten realisiert werden.

Ein weiterer, jedoch nicht beanspruchter, Gegenstand ist eine Polyaxialpedikelschraube (2) mit einem einen Gewindeschaft (6) und einen Kopf (8) aufweisenden Schraubanker (4) und mit einem in einer Seitenansicht U-förmigen und eine Aufnahmeöffnung (16) für ein Korrekturelement, insbesondere einen Korrekturstab, aufweisenden Gabelkopf (10) mit zwei Schenkeln (12),
wobei der Kopf (8) des Schraubankers (4) in einem distalen Endbereich (14) des Gabelkopfs (10) polyaxial verschwenkbar gelagert ist und der Gabelkopf (10) in einer vom Chirurgen intendierten Schwenkstellung bezüglich des Kopfs (8) des im Knochen festgelegten oder festlegbaren Schraubankers (4) fixierbar ist,
wobei der Gabelkopf (10) eine axiale Richtung (20) und eine hierzu radiale Richtung (22) aufweist sowie ein distales Ende benachbart zu dem Schraubanker (4) und ein hiervon in axialer Richtung (20) abgewandtes proximales Ende (27) aufweist, so dass auch eine distale Richtung (28) und eine proximale Richtung (29) definiert ist,
wobei sich die Schenkel (12) ausgehend von einem distalen Bereich des Gabelkopfs (10) in proximaler Richtung (29) erstrecken und proximal frei enden und zwischen sich die Aufnahmeöffnung (16) für das Korrekturelement begrenzen, wobei die Schenkel (12) einen radial äußeren Umfangsbereich aufweisen, in dem zum Ergreifen des Gabelkopfs (10) mittels eines Handhabungsinstruments wenigstens eine Haltenut oder eine sonstige Instrumentenansetzstelle ausgebildet ist,
und mit einem im Gabelkopf (10) zwischen dem Kopf (8) des Schraubankers (4) und dem Korrekturelement anordenbaren Druckstück (18), das einerseits auf dem Kopf (8) des Schraubankers (4) aufsitzt und andererseits durch das Korrekturelement belastbar ist,
wobei auf das Druckstück (18) mittels des am Gabelkopf (10) angreifenden Handhabungsinstruments oder mittels eines anderen Instruments eine temporär wirkende Kraft in Richtung auf den Kopf (8) des Schraubankers (4) ausübbar ist, so dass der Gabelkopf (10) hierdurch gegenüber dem Kopf (8) des Schraubankers (4) temporär in einer vom Chirurgen gewünschten Schwenkstellung unverstellbar gehalten ist, während das Korrekturelement bewegbar bleibt, solange bis das Korrekturelement, der Gabelkopf (10), das Druckstück (18) und der Kopf (8) des Schraubankers (4) in einer gewünschten Lage und Stellung zueinander durch ein anderes Stellmittel dauerhaft gegeneinander fixiert werden, **dadurch gekennzeichnet,** dass das Druckstück (18) im Bereich eines Schenkels (12) des Gabelkopfs (10) über ein Schwenklager (54) gegen diesen Schenkel (12) in axialer Richtung (20) abgestützt ist und dass das Druckstück (18) diametral gegenüberliegend einen Aufnahmebereich (42) für eine in axialer Richtung (20) wirkende Stellkraft (44) aufweist, welche das Druckstück (18) in distaler Richtung bezüglich des Schwenklagers (54) zu verschwenken sucht und hierdurch die temporär wirkende Kraft in Richtung auf den Kopf (8) des Schraubankers (4) bewirkt. Auch die Merkmale der abhängigen Patentansprüche werden in Verbindung mit dieser vorstehenden Pedikelschraube als zur Erfindung gehörig angesehen.

Weitere Merkmale, Vorteile und Einzelheiten der Erfindung ergeben sich aus den beigefügten Patentansprüchen und der zeichnerischen Darstellung und nachfolgenden Beschreibung bevorzugter Ausführungsformen der erfindungsgemäßen Polyaxialpedikelschraube. In der Zeichnung zeigt:
Figur 1 eine perspektivische Ansicht einer erfindungsgemäßen Ausführungsform einer Polyaxialpedikelschraube;
Figur 2 eine perspektivische Ansicht der explosionsartig dargestellten Komponenten der Pedikelschraube nach Figur 1;
Figur 3 eine Schnittansicht der Pedikelschraube nach Figur 1 mit die axiale Richtung einschließender Schnittebene;
Figur 4 die Schnittansicht nach Figur 3 mit einem die Pedikelschraube ergreifenden und eine temporäre Klemmung ausübenden Handhabungsinstrument;
Figur 5 eine Schnittansicht einer weiteren erfindungsgemäßen Ausführungsform einer Polyaxialpedikelschraube;
Figur 6 eine perspektivische Ansicht der explosionsartig dargestellten Komponenten der Pedikelschraube nach Figur 5;
Figur 7 eine Schnittansicht einer weiteren erfindungsgemäßen Ausführungsform einer Polyaxialpedikelschraube;
Figur 8 ein Detail der Figur 8;
Figur 9 eine perspektivische Ansicht einer weiteren erfindungsgemäßen Ausführungsform einer Polyaxialpedikelschraube, und

Figur 10 eine Schnittansicht der Pedikelschraube nach Figur 9 mit die axiale Richtung einschließender Schnittebene.

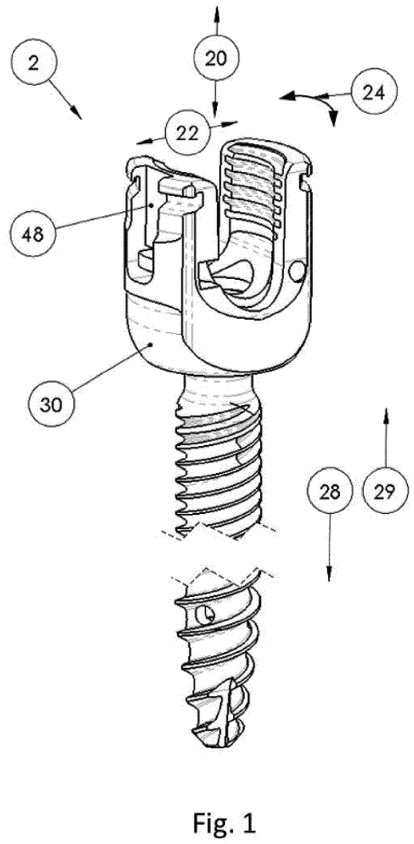

Die Figuren 1 bis 4 zeigen eine erfindungsgemäße Polyaxialpedikelschraube 2. Die Pedikelschraube 2 umfasst einen Schraubanker 4 mit Gewindeschaft 6 und Kopf 8 sowie einen Gabelkopf 10 mit zwei Schenkeln 12, die sich ausgehend von einem distalen Endbereich 14 des Gabelkopfs 10 in proximaler Richtung, also von dem Schraubanker 4 weg erstrecken und frei enden. Sie begrenzen eine U-förmige Aufnahmeöffnung 16 für ein Druckstück 18 und ein nicht dargestelltes Korrekturelement, insbesondere einen Korrekturstab, das in an sich bekannter Weise in die Aufnahmeöffnung 16 eingelegt wird und benachbarte oder gegebenenfalls mehrere Pedikelschrauben miteinander verbindet. Der Kopf 8 des Schraubankers 4 ist in dem distalen Endbereich 14 des Gabelkopfs 10 polyaxial verschwenkbar gelagert. Bei Ausübung einer hinreichenden Kraft auf das Druckstück 18 in Richtung auf den Kopf 8 kann der Gabelkopf 10 gegenüber dem Kopf 8 des Schraubankers unverstellbar gestellt werden infolge der hierdurch erzeugten Klemmwirkung, was nachfolgend noch im Einzelnen erläutert werden wird. Der Gabelkopf 10 definiert des Weiteren eine axiale Richtung 20, eine hierzu radiale Richtung 22 sowie eine Umfangsrichtung 24, ferner den distalen Endbereich 14 und einen proximalen Endbereich 26 bzw. ein proximales Ende 27. Somit ist auch eine distale Richtung 28 und eine proximale Richtung 29 gegeben.

Genau ein Schenkel 12 des Gabelkopfs 10 weist auf seiner Innenseite ausgehend von einer seitlichen Flanke des Schenkels eine Ausnehmung 30 auf, die sich in Umfangsrichtung 24 oder jedenfalls orthogonal zur axialen Richtung 20 erstreckt. Diese Ausnehmung 30 erstreckt sich weiter in radialer Richtung 22 durch einen Wandbereich 32 des Schenkels 12 hindurch und mündet im Außenumfang des Schenkels 12.

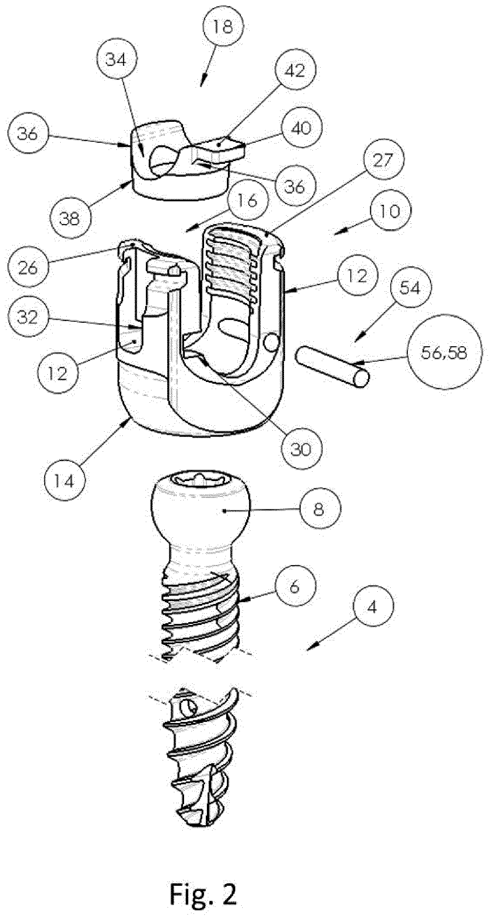

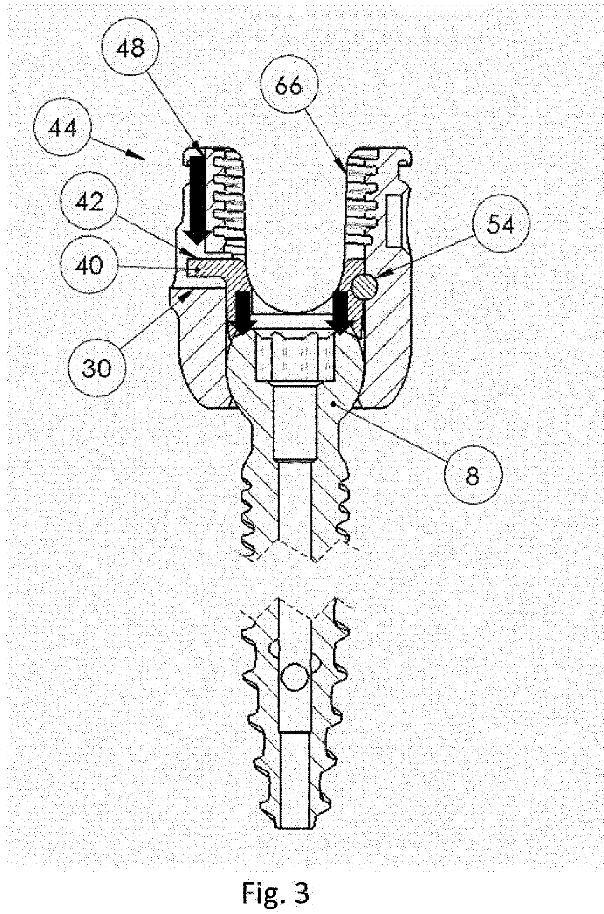

Wie man aus der Darstellung des Druckstücks 18 (am besten Figur 2) ersehen kann, umfasst das Druckstück 18 eine ungefähr halbschalenförmige Aufnahme 34 für ein stabförmiges Korrekturelement. Diese halbschalenförmige Aufnahme 34 wird seitlich begrenzt durch in distaler Richtung erstreckte Wandabschnitte 36, die einander gegenüberliegen und sich ausgehend von einem ringförmigen geschlossenen Basisabschnitt 38 des Druckstücks 18 in distaler Richtung erstrecken. Ausgehend von einem der Wandabschnitte 36 erstreckt sich ein Ansatz oder Fortsatz 40 in radialer Richtung nach außen. Dieser Ansatz oder Fortsatz 40 ist als Lasche oder Zunge ausgebildet, die sich im beispielhaft dargestellten Fall in einer Ebene orthogonal zur axialen Richtung 20 erstreckt. Ausgehend von der in Figur 2 dargestellten Orientierung des Druckstücks 18 lässt sich das Druckstück 18 in axialer Richtung 20 in den Gabelkopf 10 einführen und dann um die axiale Richtung 20 verdrehen, so dass der Ansatz oder Fortsatz 40 in die vorerwähnte in Umfangsrichtung erstreckte Ausnehmung 30 eingreifen kann. In der in Figuren 1 und 3 dargestellten Orientierung ragt der Ansatz oder Fortsatz 40 durch die im weiteren Verlauf nach radial außen öffnende Ausnehmung 30, so dass ein freies Ende des Ansatzes oder Fortsatzes 40 in axialer Richtung exponiert ist und einen Aufnahmebereich 42 für eine in axialer Richtung wirkende Stellkraft bildet, die in Figur 3 mit einem Pfeil 44 angedeutet ist.

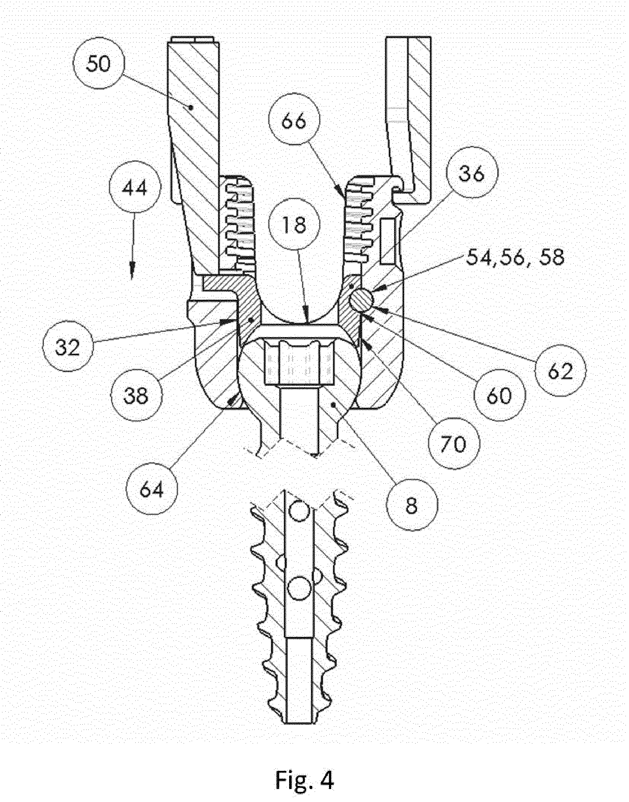

Man erkennt ferner, dass der betreffende Schenkel 12 des Gabelkopfs 10 an seiner Außenseite eine im beispielhaft dargestellten Fall nach radial außen offene und in axialer Richtung 20 erstreckte Zugangsausnehmung 48 aufweist, die einen Zugang zu der nach radial außen öffnenden Ausnehmung 30 gestattet, in der der Aufnahmebereich 42 des Ansatzes oder Fortsatzes 40 des Druckstücks 18 zu liegen kommt. Somit kann durch diese Zugangsausnehmung 48 hindurch mittels eines Stößels oder Stempels eines die Pedikelschraube haltenden Handhabungsinstruments oder eines anderen Instruments eine Stellkraft in Richtung des Pfeils 44 in Figur 3 auf den Aufnahmebereich 42 des Druckstücks 18 ausgeübt werden. Dies ist in Figur 4 dargestellt. Das Druckstück 18 ist auf seiner dem Ansatz oder Fortsatz 40 diametral gegenüberliegenden Seite so ausgebildet, dass es mit dem anderen Schenkel 12 des Gabelkopfs 10 ein Schwenklager 54 ausbildet, und zwar derart, dass das Druckstück 18 bei Einleitung einer Stellkraft in Richtung des Pfeils 44 in einer die axiale Richtung 20 einschließenden Ebene in Richtung auf den Kopf 8 des Schraubankers 4 verschwenkbar ist. Dies ist im Ausführungsbeispiel der Figuren 1 bis 4 dadurch realisiert, dass zur Ausbildung des Schwenklagers 54 ein Wellenelement 56 beispielhaft in Form eines zylindrischen Stifts 58 an der Innenseite des Schenkels 12 des Gabelkopfs 10 derart vorgesehen ist, dass es nach radial innen vorsteht, so dass das Druckstück mit einer entsprechenden Formgebung seines Wandabschnitts 36 sich daran schwenkbar abstützen kann. Hierfür weist der Wandabschnitt 36 im beispielhaft dargestellten Fall eine dem zylindrischen Stift 58 entsprechende komplementäre Ausnehmung 60 auf, wie am besten aus Figur 4 ersichtlich ist. Der zylindrische Stift 58 ist in eine Querbohrung 59 des Schenkels 12 eingesetzt, insbesondere eingepresst.

Wenn jetzt ausgehend von der in Figur 4 gezeigten Konfiguration über einen Stößel 50 eine in axialer Richtung 44 wirkende Stellkraft auf den Aufnahmebereich 42 des Ansatzes oder Fortsatzes 40 des Druckstücks 18 ausgeübt wird, so bildet das Druckstück 18 einen Hebel, der an dem Schwenklager 54 einen Schwenkpunkt 62 bildet. Da das Druckstück 18 mit seinem ringförmigen Basisabschnitt 38 auf dem Kopf 8 des Schraubankers 4 aufliegt, stützt es sich daher an der unteren, also dem Kopf 8 zugewandten Seite des Wellenelements 56 ab. Es drückt dabei mit seinem ringförmigen Basisabschnitt 38 auf den Kopf 8 des Schraubankers. Hierdurch wird der Kopf 8 des Schraubankers gegen eine kalottenartig ausgebildete Lager- oder Klemmfläche 64 im distalen Endbereich 14 des Gabelkopfs gedrückt und kann hierdurch temporär lagefixiert, d.h. temporär unverstellbar, gestellt werden, solange die Stellkraft auf das Druckstück 18 ausgeübt wird. Währenddessen hat der Chirurg Zeit, das in den Figuren nicht dargestellte Korrekturelement innerhalb des Gabelkopfs zu positionieren und Lage- und Orientierungsänderungen des Wirbels, in welchen der Schraubanker 4 eingeschraubt ist, vorzunehmen. Schließlich können alle Komponenten in einer gewünschten Orientierung zueinander beispielsweise dadurch fixiert werden, dass in ein Innengewinde 66 an den Schenkeln 12 des Gabelkopfs 10 eine nicht dargestellte Madenschraube eingeschraubt wird, die dann auf das Korrekturelement drückt, welches wiederum in der halbschalenförmigen Aufnahme 34 des Druckstücks 18 aufliegt und diese Druckkraft auf den Kopf 8 des Schraubankers weitergibt. Nach Fixierung dieser Madenschraube kann der Stößel 50 des Handhabungsinstruments gelöst werden.

Man erkennt ferner aus Figuren 3 und 4, dass zwischen dem Gabelkopf 10 und dem ringförmigen Basisabschnitt 38 des Druckstücks ein Umfangsspalt 70 ausgebildet ist, der sich in proximaler Richtung verengt. Auf diese Weise ist genug Raum zur Verfügung gestellt, damit das Druckstück 18 verschwenken kann.

Des Weiteren erweist es sich als vorteilhaft, wenn die Ausbildung des Schwenklagers 54 derart ist, dass das Druckstück 18 mit seiner dem Wellenelement 56 zugewandten Seite in axialer Richtung 20 spielbehaftet mit dem Wellenelement 56 zusammenwirkt. Es wurde schon darauf hingewiesen, dass sich das Druckstück 18 von unten gegen das Wellenelement 56 abstützt. Es erweist sich daher als vorteilhaft, wenn die Ausnehmung 60, welche das Wellenelement 56 umgibt, in axialer Richtung 20 größer dimensioniert ist als das Wellenelement 56 im Bereich der Ausnehmung 60. Dieses axiale Spiel hat zur Folge, dass sich das Druckstück 18 bei der schlussendlichen Fixierung, wenn das Korrekturelement auf die halbschalenförmige Aufnahme 34 in axialer Richtung 20 gedrückt wird, dann symmetrisch in Richtung auf den Kopf 8 verlagern und verspannen kann, ohne dass es zu Verwindungen infolge des Schwenklagers kommt.

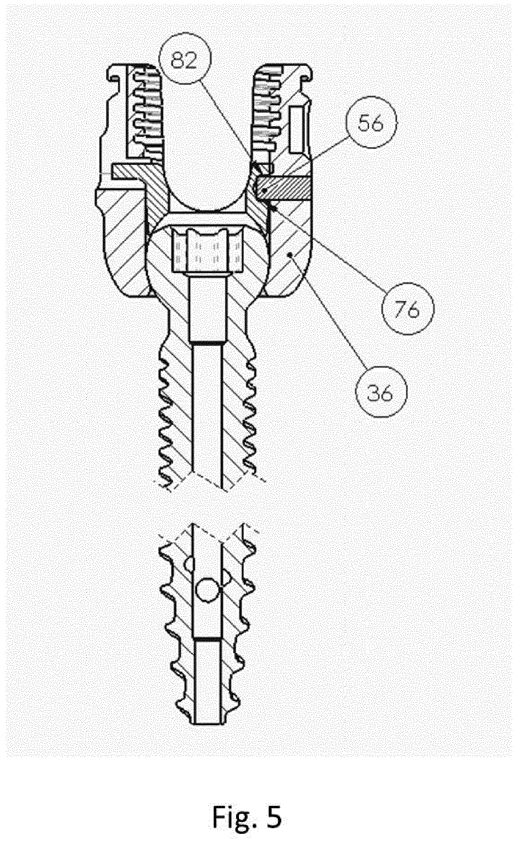

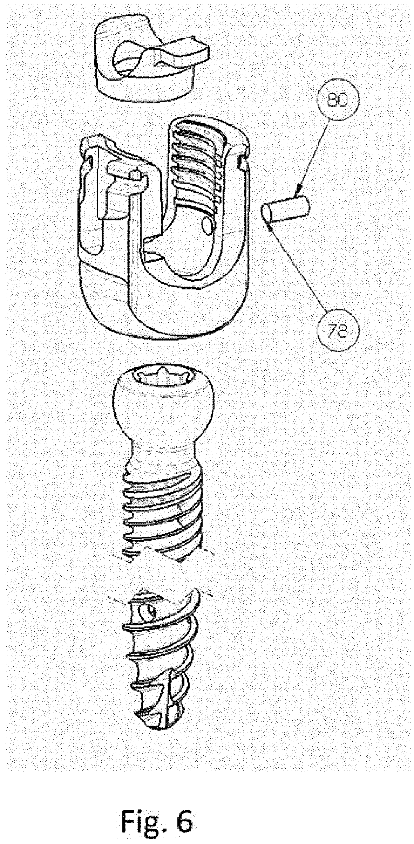

Figuren 5 und 6 zeigen ein weiteres Ausführungsbeispiel, wonach das Schwenklager 56 ein radial innen am Gabelkopf 10 ausgebildetes Auflager 76 umfasst. Dieses Auflager 76 ist von einem freien Ende 78 eines von außen nach radial innen durch den betreffenden Schenkel 12 des Gabelkopfs 10 hindurchgesteckten Stifts 80 gebildet. Zur Aufnahme des freien Endes 78 weist der betreffende Wandabschnitt 36 des Druckstücks 18 eine nach radial außen öffnende Ausnehmung 82 auf, die derart dimensioniert und spielbehaftet gegenüber dem freien Ende 78 des Stifts 80 ausgebildet ist, dass das Druckstück in einer die axiale Richtung 20 einschließenden Ebene verschwenken kann, so wie dies bei der vorausgehend beschriebenen Ausführungsform der Fall ist.

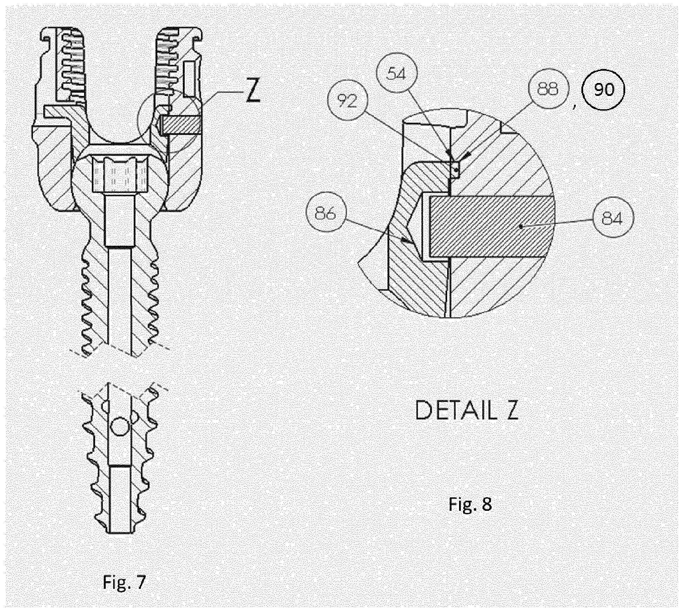

Eine weitere Ausführungsform ist in Figuren 7 und 8 dargestellt. Bei dieser Ausführungsform ist auch ein Stift 84 vorgesehen, der in eine Öffnung 86 des Druckstücks 18 eingreift; er ist aber lediglich als in Umfangsrichtung wirkende Verdrehsicherung konzipiert. Das Schwenklager 54 ist in diesem Fall von einer einstückig mit dem Gabelkopf ausgebildeten Nutflanke 88, die ein Auflager 90 bildet, realisiert, wobei der Wandabschnitt 36 des Druckstücks einen nach radial außen erstreckten ein Gegenlager bildenden Stützabschnitt 92 aufweist, der in die Nut eingreift und sich axial gegen die Nutflanke 88 abstützt.

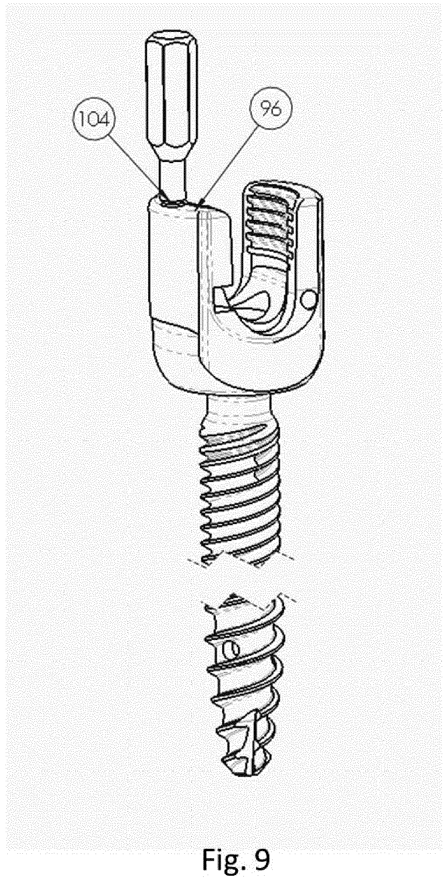

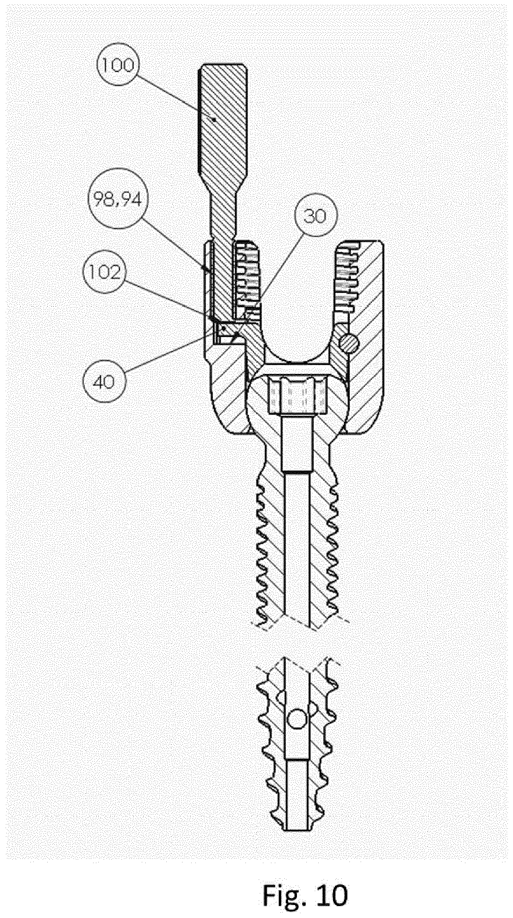

Schließlich zeigen die Figuren 9 und 10 eine weitere Ausführungsform, wonach eine Zugangsausnehmung 94 ausgehend von einem proximalen Ende 96 des zugeordneten Schenkels 12 als axiale Bohrung 98 ausgebildet ist. Diese axiale Bohrung 98 mündet in der Ausnehmung 30, durch die sich der Fortsatz 40 des Druckstücks 18 erstreckt. Im beispielhaften Fall trägt die axiale Bohrung 98 ein Innengewinde, in welche ein Gewindestößel 100 einschraubbar ist, bis er mit seinem distalen Ende 102 gegen den Fortsatz 40 des Druckstücks anliegt und dieses in Richtung auf den Kopf 8 des Schraubankers zu verschwenken sucht. Es wäre auch denkbar, dass dieser Gewindestößel 100 im Bereich des proximalen Endes 96 des Gabelkopfs 10 eine Sollbruchstelle 104 umfasst, an der der Gewindestößel 100 abscherbar ist.

## Patentansprüche

1. Polyaxialpedikelschraube (2) mit einem einen Gewindeschaft (6) und einen Kopf (8) aufweisenden Schraubanker (4) und mit einem in einer Seitenansicht U-förmigen und eine Aufnahmeöffnung (16) für ein Korrekturelement, insbesondere einen Korrekturstab, aufweisenden Gabelkopf (10) mit zwei Schenkeln (12), wobei der Kopf (8) des Schraubankers (4) in einem distalen Endbereich (14) des Gabelkopfs (10) polyaxial verschwenkbar gelagert ist und der Gabelkopf (10) in einer vom Chirurgen intendierten Schwenkstellung bezüglich des Kopfs (8) des im Knochen festgelegten oder festlegbaren Schraubankers (4) fixierbar ist,
wobei der Gabelkopf (10) eine axiale Richtung (20) und eine hierzu radiale Richtung (22) aufweist sowie ein distales Ende benachbart zu dem Schraubanker (4) und ein hiervon in axialer Richtung (20) abgewandtes proximales Ende (27) aufweist, so dass auch eine distale Richtung (28) und eine proximale Richtung (29) definiert ist, wobei sich die Schenkel (12) ausgehend von einem distalen Bereich des Gabelkopfs (10) in proximaler Richtung (29) erstrecken und proximal frei enden und zwischen sich die Aufnahmeöffnung (16) für das Korrekturelement begrenzen, wobei die Schenkel (12 ) einen radial äußeren Umfangsbereich aufweisen, in dem zum Ergreifen des Gabelkopfs (10) mittels eines Handhabungsinstruments wenigstens eine Haltenut oder eine sonstige Instrumentenansetzstelle ausgebildet ist,
und mit einem im Gabelkopf (10) zwischen dem Kopf (8) des Schraubankers (4) und dem Korrekturelement anordenbaren Druckstück (18), das einerseits auf dem Kopf (8) des Schraubankers (4) aufsitzt und andererseits durch das Korrekturelement belastbar ist,
wobei auf das Druckstück (18) mittels des am Gabelkopf (10) angreifenden Handhabungsinstruments oder mittels eines anderen Instruments eine temporär wirkende Kraft in Richtung auf den Kopf (8) des Schraubankers (4) ausübbar ist, so dass der Gabelkopf (10) hierdurch gegenüber dem Kopf (8) des Schraubankers (4) temporär in einer vom Chirurgen gewünschten Schwenkstellung unverstellbar gehalten ist, während das Korrekturelement bewegbar bleibt, solange bis das Korrekturelement, der Gabelkopf (10), das Druckstück (18) und der Kopf (8) des Schraubankers (4) in einer gewünschten Lage und Stellung zueinander durch ein anderes Stellmittel dauerhaft gegeneinander fixiert werden, wobei das Druckstück (18) im Bereich eines Schenkels (12) des Gabelkopfs (10) über ein Schwenklager (54) gegen diesen Schenkel (12) in axialer Richtung (20) abgestützt ist, **dadurch gekennzeichnet, dass** eine in axialer Richtung (20) wirkende Stellkraft (44) nur an einer einzigen Stelle dem Schwenklager (54) diametral gegenüberliegend in das Druckstück (18) eingeleitet wird, indem das Druckstück (18) dem Schwenklager (54) diametral gegenüberliegend einen Aufnahmebereich (42) für die in axialer Richtung (20) wirkende Stellkraft (44) aufweist, welche das Druckstück (18) in distaler Richtung bezüglich des Schwenklagers (54) zu verschwenken sucht und hierdurch die temporär wirkende Kraft in Richtung auf den Kopf (8) des Schraubankers (4) bewirkt, und dass der Gabelkopf (10) hierfür eine Zugangsausnehmung (48) in genau einem der Schenkel (12) aufweist, durch welche Zugang auf den Aufnahmebereich (42) des Druckstücks (18) mittels des Handhabungsinstruments oder des anderen Instruments genommen werden kann.

2. Pedikelschraube nach Anspruch 1, **dadurch gekennzeichnet, dass** das Schwenklager (54) ein in den Gabelkopf (10) eingesetzes langgestrecktes Wellenelement (56), insbesondere einen Stift (58), aufweist, das in einer Ebene orthogonal zur axialen Richtung (20) des Gabelkopfs (10) erstreckt ist und gegen welches das Druckstück (18) in axialer Richtung (20) abgestützt ist.

3. Pedikelschraube nach Anspruch 1 oder 2, **dadurch gekennzeichnet, dass** das langgestreckte Wellenelement (56) ungefähr parallel zu dem in den Gabelkopf eingesetzten Korrekturelement erstreckt ist.

4. Pedikelschraube nach einem oder mehreren der vorstehenden Ansprüche, **dadurch gekennzeichnet, dass** das Schwenklager (54) ein radial innen am Gabelkopf (10) ausgebildetes Auflager (76, 90) umfasst, gegen welches das Druckstück (18) mit einem Gegenlager in axialer Richtung (20) abgestützt ist.

5. Pedikelschraube nach Anspruch 4, **dadurch gekennzeichnet, dass** das Auflager (76) von einem freien Ende (78) eines von außen nach radial innen durch einen Schenkel (12) des Gabelkopfs (10) gesteckten Stifts (80) gebildet ist.

6. Pedikelschraube nach Anspruch 4, **dadurch gekennzeichnet, dass** das Auflager (90) von einer am Gabelkopf (10), insbesondere einstückig mit dem Gabelkopf ausgebildeten Stufe oder Nutflanke (88) gebildet ist.

7. Pedikelschraube nach einem oder mehreren der vorstehenden Ansprüche, **dadurch gekennzeichnet, dass** das Schwenklager (54) eine Ausnehmung (60, 82) in dem Druckstück (18) umfasst.

8. Pedikelschraube nach einem oder mehreren der vorstehenden Ansprüche, **dadurch gekennzeichnet, dass** das Schwenklager (54) einen radial vorstehenden Stützabschnitt (92) an dem Druckstück (18) umfasst, mittels dessen das Druckstück (18) am Gabelkopf (10) schwenkbar abgestützt ist.

9. Pedikelschraube nach einem oder mehreren der vorstehenden Ansprüche, **dadurch gekennzeichnet, dass** der Aufnahmebereich (42) des Druckstücks (18) für die in axialer Richtung (20) wirkende Stellkraft (44) von einem radialen Ansatz oder Fortsatz (40) des Druckstücks (18) gebildet ist.

10. Pedikelschraube nach einem oder mehreren der vorstehenden Ansprüche, **dadurch gekennzeichnet, dass** der Gabelkopf (10) eine Ausnehmung (30) für den Aufnahmebereich (42) des Druckstücks (18) aufweist.

11. Pedikelschraube nach Anspruch 10, **dadurch gekennzeichnet, dass** die Ausnehmung (30) in axialer Richtung (20) oder in Umfangsrichtung (24) oder quer zur axialen Richtung (20) an einem Schenkel (12) des Gabelkopfs (10)jeweils innen erstreckt ist und jeweils nach innen öffnend ausgebildet ist.

12. Pedikelschraube nach Anspruch 10, **dadurch gekennzeichnet, dass** die Ausnehmung (30) in radialer Richtung (22) durch einen Wandbereich eines Schenkels (12) des Gabelkopfs (10) hindurchgehend ausgebildet und erstreckt ist.

13. Pedikelschraube nach Anspruch 12, **dadurch gekennzeichnet, dass** die Ausnehmung (30) ausgehend von einer Flanke des Schenkels (12) des Gabelkopfs (10) zuerst in Umfangsrichtung (24) oder quer zur axialen Richtung (20) innen an dem Schenkel und dann in radialer Richtung (22) durch einen Wandbereich (32) des Schenkels hindurchgehend ausgebildet und erstreckt ist, und insbesondere in der Außenumfangsfläche ausmündet.

14. Pedikelschraube nach einem oder mehreren der vorstehenden Ansprüche, **dadurch gekennzeichnet, dass** die Zugangsausnehmung (48) ausgehend von einem proximalen Ende des Schenkels (12) des Gabelkopfs (10) in axialer Richtung (20) erstreckt ist und dass die Zugangsausnehmung (48) dabei radial innen ausgebildet ist und nach radial innen offen ist oder radial außen ausgebildet ist und nach radial außen offen ist oder als axiale Bohrung (98) in dem Schenkel ausgebildet ist.

15. Pedikelschraube nach einem oder mehreren der vorstehenden Ansprüche, **dadurch gekennzeichnet, dass** das Schwenklager (54) ein axiales Spiel aufweist, derart, dass das Druckstück (18) bei Krafteinleitung in axialer Richtung (20) über das Korrekturelement eine axiale Stellbewegung ausführen kann, ohne dass diese Stellbewegung durch das Schwenklager (54) behindert würde.

## Claims

1. A polyaxial pedicle screw (2) comprising a screw anchor (4), which has a threaded shaft (6) and a head (8), and comprising a fork head (10), which is U-shaped in side view and has a receiving opening (16) for a corrective element, in particular a correcting rod, and two arms (12),
wherein the head (8) of the screw anchor (4) is polyaxially pivotably mounted in a distal end region (14) of the fork head (10) and the fork head (10) can be fixed in a pivoted position intended by the surgeon relative to the head (8) of the screw anchor (4) that is fixed or can be fixed in the bone,
wherein the fork head (10) has an axial direction (20) and a direction (22) radial to this as well as a distal end adjacent to the screw anchor (4) and has a proximal end (27) facing away from this in the axial direction (20), so that a distal direction (28) and a proximal direction (29) are defined as well,
wherein the arms (12) starting from a distal region of the fork head (10) extend in the proximal direction (29) and terminate with proximally free ends and delimit the receiving opening (16) for the corrective element between them,
wherein the arms (12) have a radially outer peripheral region, in which at least one retaining groove or other instrument placement location is formed in order to engage the fork head (10) via a handling instrument, and comprising a pressure piece (18) which can be arranged in the fork head (10) between the head (8) of the screw anchor (4) and the corrective element which, on the one hand, rests on the head (8) of the screw anchor (4) and, on the other hand, can be loaded by the corrective element,
wherein a force can be exerted temporarily on the pressure piece (18) in the direction of the head (8) of the screw anchor (4) via the handling instrument engaging on the fork head (10) or via another instrument so that the fork head (10) is temporarily fixed by this in a pivoted position intended by the surgeon relative to the head (8) of the screw anchor (4), whereas the corrective element remains movable as long as the corrective element, the fork head (10), the pressure piece (18) and the head (8) of the screw anchor (4) are durably fixed in a desired position and orientation with respect to one another via another adjusting means, wherein the pressure piece (18) in the region of an arm (12) of the fork head (10) is supported by means of a pivot bearing (54) against this arm (12) in the axial direction (20) and
**characterized in that** a positioning force (44) acting in the axial direction (20) is exerted onto the pressure piece (18) at only one single place diametrically opposite the pivot bearing (54), by the pressure piece (18) having a receiving region (42) diametrically opposite the pivot bearing (54) for the positioning force (44) acting in the axial direction (20) so as to pivot the pressure piece (18) in the distal direction relative to the pivot bearing (54) and thereby exerting the temporarily acting force in the direction of the head (8) of the screw anchor (4), and
that the fork head (10) has an access recess (48) in precisely one of the arms (12), through which the receiving region of the pressure piece (18) can be accessed via the handling instrument or another instrument.

2. Pedicle screw according to claim 1, **characterized in that** the pivot bearing (54) has an elongated shaft element (56) inserted into the fork head (10) that extends in a level orthogonal to the axial direction (20) of the fork head (10) and against which the pressure piece (18) is supported in the axial direction (20).

3. Pedicle screw according to claim 1 or 2, **characterized in that** the elongated shaft element (56) extends roughly parallel to the corrective element inserted into the fork head.

4. Pedicle screw according to any one or more of the preceding claims, **characterized in that** the pivot bearing (54) comprises a support (76, 90) formed radially to the inside on the fork head (10), against which the pressure piece (18) is supported in the axial direction (20) by a counter support.

5. Pedicle screw according to claim 4, **characterized in that** the support (76) is formed by a free end (78) of a pin (80) inserted from the outside radially inward through an arm (12) of the fork head (10).

6. Pedicle screw according to claim 4, **characterized in that** the support (90) is formed by a step or groove flank (88) on the fork head (10), in particular formed as one piece with the fork head (10).

7. Pedicle screw according to any one or more of the preceding claims, **characterized in that** the pivot bearing (54) comprises a recess (60, 82) in the pressure piece (18) .

8. Pedicle screw according to any one or more of the preceding claims, **characterized in that** the pivot bearing (54) comprises a radially projecting support section (92) on the pressure piece (18), by means of which the pressure piece (18) is pivotably supported on the fork head (10).

9. Pedicle screw according to any one or more of the preceding claims, **characterized in that** the receiving region (42) of the pressure piece (18) for the positioning force (44) acting in the axial direction (20) is formed by a radial projection or extension (40) of the pressure piece (18).

10. Pedicle screw according to any one or more of the preceding claims, **characterized in that** the fork head (10) has a recess (30) for the receiving region (42) of the pressure piece (18).

11. Pedicle screw according to claim 10, **characterized in that** the recess (30) in the axial direction (20) or in a circumferential direction (24) or transverse to the axial direction (20) is respectively extended radially inward on an arm (12) of the fork head (10) and is respectively formed opening inwardly.

12. Pedicle screw according to claim 10, **characterized in that** the recess (30) is formed and extended in the radial direction (22) through a wall region of an arm (12) of the fork head (10).

13. Pedicle screw according to claim 12, **characterized in that** the recess (30) is formed beginning from a flank of the arm (12) of the fork head (10) on the inside of the arm and extends first inward in the circumferential direction (24) or transverse to the axial direction (20) and then in the radial direction (22) through a wall region (32) of the arm and in particular opens out in the outer circumference surface.

14. Pedicle screw according to one or more of the preceding claims **characterized in that** the access recess (48) extends in the axial direction (20) beginning from a proximal end of the arm (12) of the fork head (10) and that the access recess (48) is formed radially inside und is open to radially inside or is formed radially outside and is open to radially outside or is formed a an axial bore (98) within the arm.

15. Pedicle screw according to any one or more of the preceding claims, **characterized in that** the pivot bearing (54) has an axial play such that, upon introduction of force in the axial direction (20) via the corrective element, the pressure piece (18) can perform an axial adjustment movement without this adjustment movement being hindered by the pivot bearing (54).

## Revendications

1. Vis pédiculaire polyaxiale (2) avec un ancrage à vis (4) présentant une tige filetée (6) et une tête (8) et avec une chape (10), avec deux branches (12), en forme de U en vue latérale et présentant une ouverture de réception (16) pour un élément de correction, en particulier une barre de correction,
dans laquelle la tête (8) de l'ancrage à vis (4) est montée pivotante de manière polyaxiale dans une zone d'extrémité distale (14) de la chape (10) et la chape (10) peut être fixée, dans une position de pivotement prévue par le chirurgien, par rapport à la tête (8) de l'ancrage à vis (4) bloqué ou pouvant être bloqué dans l'os,
dans laquelle la chape (10) présente une direction axiale (20) et une direction radiale (22) par rapport à celle-ci ainsi qu'une extrémité distale au voisinage de l'ancrage à vis (4) et une extrémité proximale (27) opposée à celle-ci dans la direction axiale (20), de sorte qu'une direction distale (28) et une direction proximale (29) est également définie,
dans laquelle les branches (12) s'étendent à partir d'une zone distale de la chape (10) dans la direction proximale (29) et terminent librement de manière proximale et délimitent entre elles l'ouverture de réception (16) pour l'élément de correction,
dans laquelle les branches (12) présentent une zone périphérique radialement extérieure, dans laquelle est réalisé(e) au moins une rainure de retenue ou un autre point d'application d'instrument pour la saisie de la chape (10) au moyen d'un instrument de manipulation,
et avec une pièce de poussée (18) pouvant être agencée dans la chape (10) entre la tête (8) de l'ancrage à vis (4) et l'élément de correction, qui repose d'une part sur la tête (8) de l'ancrage à vis (4) et peut d'autre part être sollicitée par l'élément de correction,
dans laquelle une force agissant temporairement peut être exercée en direction de la tête (8) de l'ancrage à vis (4) sur la pièce de poussée (18) au moyen de l'instrument de manipulation agissant sur la chape (10) ou au moyen d'un autre instrument, de sorte que la chape (10) est ainsi retenue temporairement de manière immobile dans une position de pivotement souhaitée par le chirurgien par rapport à la tête (8) de l'ancrage à vis (4), pendant que l'élément de correction reste mobile, jusqu'à ce que l'élément de correction, la chape (10), la pièce de poussée (18) et la tête (8) de l'ancrage à vis (4) soient fixés durablement les uns par rapport aux autres dans une position souhaitée par un autre moyen de réglage, dans laquelle la pièce de poussée (18) est en appui dans la zone d'une branche (12) de la chape (10) par l'intermédiaire d'un palier pivotant (54) contre cette branche (12) dans la direction axiale (20),
**caractérisée en ce que** une force de réglage (44) agissant dans la direction axiale (20) est exercée à un seul endroit diamétralement opposée au palier pivotant (54) sur la pièce de poussée (18), par la pièce de poussée (18) présentent une zone de réception (42) pour la force de réglage (44) agissant dans la direction axiale (20), cette zone de réception (42) étant située diamétralement opposée au palier pivotant (54), la force de réglage (44) cherche à faire pivoter la pièce de poussée (18) dans la direction distale par rapport au palier pivotant (54) et provoque ainsi la force agissant temporairement en direction de la tête (8) de l'ancrage à vis (4), et **en ce que** la chape (10) présente un évidement d'accès (48) dans une seule des branches (12), par lequel l'accès à la zone de réception de la pièce de poussée (18) au moyen de l'instrument de manipulation ou de l'autre instrument peut être donné.

2. Vis pédiculaire selon la revendication 1, **caractérisée en ce que** le palier pivotant (54) présente un élément ondulé (56) étendu de tout son long inséré dans la chape (10), qui est étendu dans un plan perpendiculairement à la direction axiale (20) de la chape (10) et contre lequel la pièce de poussée (18) est en appui dans la direction axiale (20).

3. Vis pédiculaire selon la revendication 1 ou 2, **caractérisée en ce que** l'élément ondulé (56) étendu de tout son long est étendu à peu près parallèlement à l'élément de correction inséré dans la chape.

4. Vis pédiculaire selon l'une ou plusieurs des revendications précédentes, **caractérisée en ce que** le palier pivotant (54) comprend un support (76, 90) réalisé radialement à l'intérieur sur la chape (10), contre lequel la pièce de poussée (18) est en appui dans la direction axiale (20) à l'aide d'un support complémentaire.

5. Vis pédiculaire selon la revendication 4, **caractérisée en ce que** le support (76) est formé par une extrémité libre (78) d'une tige (80) introduite de l'extérieur vers radialement vers l'intérieur à travers une branche (12) de la chape (10).

6. Vis pédiculaire selon la revendication 4, **caractérisée en ce que** le support (90) est formé par un étage ou flanc de rainure (88) réalisé sur la chape (10), en particulier d'une seule pièce avec la chape.

7. Vis pédiculaire selon l'une ou plusieurs des revendications précédentes, **caractérisée en ce que** le palier pivotant (54) comprend un évidement (60, 82) dans la pièce de poussée (18).

8. Vis pédiculaire selon l'une ou plusieurs des revendications précédentes, **caractérisée en ce que** le palier pivotant (54) comprend sur la pièce de poussée (18) une partie d'appui (92) radialement en saillie, au moyen de laquelle la pièce de poussée (18) est en appui pivotant sur la chape (10).

9. Vis pédiculaire selon l'une ou plusieurs des revendications précédentes, **caractérisée en ce que** la zone de réception (42) de la pièce de poussée (18) pour la force de réglage (44) agissant dans la direction axiale (20) est formée par une saillie ou partie saillante radiale (40) de la pièce de poussée (18).

10. Vis pédiculaire selon l'une ou plusieurs des revendications précédentes, **caractérisée en ce que** la chape (10) présente un évidement (30) pour la zone de réception (42) de la pièce de poussée (18).

11. Vis pédiculaire selon la revendication 10, **caractérisée en ce que** l'évidement (30) est étendu dans la direction axiale (20) ou dans la direction périphérique (24) ou transversalement à la direction axiale (20) sur une branche (12) de la chape (10) respectivement à l'intérieur et est réalisée de manière à s'ouvrir respectivement vers l'intérieur.

12. Vis pédiculaire selon la revendication 10, **caractérisée en ce que** l'évidement (30) est réalisé et étendu dans la direction radiale (22) de manière à traverser une zone de paroi d'une branche (12) de la chape (10).

13. Vis pédiculaire selon la revendication 12, **caractérisée en ce que** l'évidement (30) est réalisé et étendu à partir d'un flanc de la branche (12) de la chape (10) tout d'abord dans la direction périphérique (24) ou transversalement à la direction axiale (20) à l'intérieur sur la branche puis dans la direction radiale (22) de manière à traverser une zone de paroi (32) de la branche, en particulier débouche dans la zone périphérique extérieure.

14. Vis pédiculaire selon l'une ou plusieurs des revendications précédentes, **caractérisée en ce que** l'évidement d'accès (48) est étendu à partir d'une extrémité proximale de la branche (12) de la chape (10) dans la direction axiale (20) et **en ce que** l'évidement d'accès (48) est formé radialement a l'intérieur et est ouvert radialement a l'intérieur ou est formé radialement a l'extérieur et est ouvert radialement a l'extérieur ou est réalisé sous la forme d'un trou axial (98) dans la branche.

15. Vis pédiculaire selon l'une ou plusieurs des revendications précédentes, **caractérisée en ce que** le palier pivotant (54) présente un jeu axial, de telle sorte que la pièce de poussée (18), lorsqu'une force est appliquée dans la direction axiale (20) par l'intermédiaire de l'élément de correction, peut exécuter un mouvement de réglage axial, sans que ce mouvement de réglage ne soit entravé par le palier pivotant (54).
